Europäisches Patentamt

European Patent Office

Office européen des brevets

(19)

(11) Veröffentlichungsnummer: **0 162 358**
**B1**

(12) **EUROPÄISCHE PATENTSCHRIFT**

(45) Veröffentlichungstag der Patentschrift: **08.08.90**

(21) Anmeldenummer: **85105450.2**

(22) Anmeldetag: **04.05.85**

(51) Int. Cl.⁵: **A 61 M 1/06**

(54) Muttermilchabsaugvorrichtung.

(30) Priorität: **25.05.84 DE 3419613**

(43) Veröffentlichungstag der Anmeldung:
**27.11.85 Patentblatt 85/48**

(45) Bekanntmachung des Hinweises auf die
Patenterteilung:
**08.08.90 Patentblatt 90/32**

(84) Benannte Vertragsstaaten:
**AT FR GB**

(56) Entgegenhaltungen:
**FR-A- 552 321**
**US-A-1 509 226**

(73) Patentinhaber: **KaWeCo GmbH Fabrik
medizinischer Apparate
Gerlinger Strasse 36-38
D-7257 Ditzingen (DE)**

(72) Erfinder: **Kirchner, Hansjörg
Paulinenstrasse 25
D-7145 Markgröningen (DE)**

(74) Vertreter: **Birn, Gerhard A. et al
Patentanwälte Dipl.-Ing. Berthold Schmid Dr.-
Ing. Gerhard Birn Dipl.-Phys. Heinrich Quarder
Falbenhennenstrasse 1
D-7000 Stuttgart 1 (DE)**

Courier Press, Leamington Spa, England.

EP 0 162 358 B1

## Beschreibung

Die Erfindung bezieht sich auf eine Muttermilchabsaugvorrichtung bestehend aus einem Trichter und einer daran angeschlossenen, mittels einer Handhabe betätigbaren Kolbenpumpe, mit an einer Kolbenstange gehaltenem Kolben sowie einem Entlüftungsventil und einem in der der Kolbenstange abgewandten Stirnfläche des Pumpenzylinders befindlichen Belüftungsventils, wobei beim Ansaughub des Kolbens das Entlüftungsventil schließt und das Belüftungsventil öffnet, während beim Rückhub des Kolbens das Entlüftungsventil öffnet und das Beflüftungsventil schließt. Eine derartige Muttermilchabsaugvorrichtung ist durch die US—A—1 509 226 bekannt geworden. Diese Vorrichtung ist nicht nur wegen ihres kleinen Auffanggefäßes, sondern auch wegen des bei handbetätigten Absaugvorrichtungen verhältnismäßig kleinen Kolbenhubs und auch des aus Gewichtsgründen kleinen Kolbendurchmessers nur zum Absaugen verhältnismäßig kleiner Milchmengen geeignet. Sollen größere Milchmengen abgesaugt werden, so muß man statt dessen eine motorbetriebene Milchabsaugvorrichtung nehmen. Ein Anschluß dieser Muttermilchabsaugvorrichtung an eine Saugpumpe ist aber nicht möglich.

Durch die FR—A—552 321 ist eine sehr einfache, ebenfalls von Hand zu betätigende Muttermilchabsaugvorrichtung bekannt geworden. Bei einer Variante dieser Vorrichtung ist eine mittels eines Stopfens außen verschließbare hohle Kolbenstange vorgesehen. Sie verfügt jedoch über keinerlei Ventile, weswegen ein theoretisch denkbares Evakuieren des Trichters über diese Kolbenstange schon allein aus gesundheitlichen Gründen völlig ausgeschlossen ist.

Die Aufgabe der Erfindung besteht nun darin, eine Muttermilchabsaugvorrichtung der eingangs beschriebenen Art so weiterzubilden, daß sie bei möglichst gleichbleibendem Unterdruck sowohl als handbetätigte als auch als motorisch betätigte Vorrichtung betrieben werden kann.

Zur Lösung dieser Aufgabe wird erfindungsgemäß vorgeschlagen, daß die Muttermilchabsaugvorrichtung gemäß dem Oberbegriff des Anspruchs 1 entsprechend dem kennzeichenden Teil dieses Anspruchs ausgebildet ist. Weil die einen Entlüftungskanal bildende hohle Kolbenstange an der Mündung des Entlüftungskanals in den Ansaugraum erfindungsgemäß das Entlüftungsventil trägt und dieses beim Ansaug-Bewegungshub des Kolbens schließt, kann man sich die Arbeitsweise dieses Rückschlagventils in vorteilhafter Weise dadurch zunutze machen, daß man am äußeren Ende des Entlüftungskanals eine Motorpumpe anschließt, deren Unterdruck ein Öffnen des Entlüftungsventils bewirkt. Man erhält dadurch auch im von der Kolbenstange angewandten Zylinderraum einen Unterdruck, der in gleicher Weise wie beim Handbetrieb öffnend auf das Belüftungsventil einwirkt. Dadurch ist ein durchgehender Unterdruck vom äußeren freien Ende der Kolbenstange bis zum Trichter möglich. Zum einen gestattet dies, innerhalb kurzer Zeit relativ viel Milch anzusaugen und zum anderen entfällt das beim Pumpen von Hand teilweise als unangenehm empfundene Einwirken der Vorrichtung über den Trichter auf die weibliche Brust.

Der konstante Unterdruck wird auch insofern als angenehm empfunden, als das ständige Ansteigen und Absenken des Unterdrucks beim Handbetrieb gleichfalls als lästig angesehen wird. Ein Umbau der Vorrichtung ist nicht möglich, vielmehr verbindet man einfach das freie Ende der Kolbenstange beispielsweise über einen Schlauch mit der Saugpumpe.

Eine weitere Ausgestaltung der Erfindung sieht vor, daß die Kolbenstange mit dem einen Ende eines zweiarmigen, schwenkbar gelagerten Hebels gelenkig verbunden ist, dessen anderes Ende als Handhabe ausgebildet ist. Dadurch wird beim Ansaugvorgang, im Gegensatz zur eingangs beschriebenen Vorrichtung, der Trichter auf die Brust aufgedrückt und nicht von dieser weggezogen, so daß stets eine dichte Anlage gewährleistet bleibt.

Zweckmäßigerweise ist das nach außen geführte Ende der Kolbenstange als Aufsteckhülse für den Saugschlauch einer Motorpumpe ausgebildet und ragt über die Anlenkstelle des Hebels hinaus. Dadurch kann der Schlauch einer Motorpumpe ohne Schwierigkeiten angeschlossen werden und vor allem, ohne daß der Betätigungshebel störend wirkt.

Gemäß einer weiteren Variante der Erfindung ist der als Handhabe ausgebildete Hebelteil an seinem freien Ende als Stütze so lang ausgebildet, daß er in mindestens einer Stellung etwa in einer Ebene mit dem Boden des Milchauffangbehälters liegt. Die Absaugvorrichtung kann infolgedessen auf einem Tisch od. dgl. abgestellt werden, ohne daß die Gefahr des Umfallens besteht.

Eine weitere bevorzugte Ausführungsform der Muttermilchabsaugvorrichtung mit einem Ventil zwischen dem Trichter und der Stirnfläche des Pumpenzylinders ist dadurch gekennzeichnet, daß das Ventil am Trichter angebracht und insbesondere als einstellbares Überdruckventil ausgebildet ist. Es verhindert, daß der Unterdruck im Trichter ein vorgegebenes Maß überschreitet, welches nicht nur unangenehm, sondern auch gesundheitsbeeinträchtigend sein könnte.

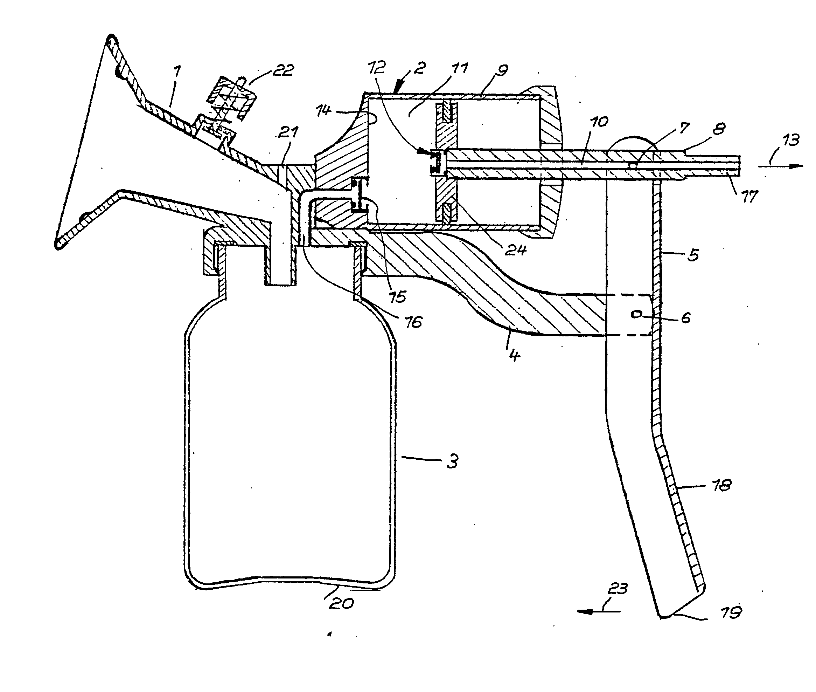

Die Zeichnung zeigt ein Ausführungsbeispiel der Erfindung anhand eines Vertikalschnitts durch die Muttermilchabsaugvorrichtung mit Milchauffangbehälter.

Der auf eine nicht dargestellte Brust aufsetzbare Trichter 1 ist mit einer Kolbenpumpe 2 verbunden, und das Ganze ist auf den Milchauffangbehälter 3 aufgeschraubt. Unterhalb der Kolbenpumpe 2 befindet sich ein nach hinten ragender Tragarm 4, an dem ein zweiarmiger Hebel 5 bei 6 schwenkbar gelagert ist. Dieser ist an seinem oberen Ende bei 7 gelenkig mit der Kolbenstange 8 verbunden, welche aus dem Zylinder 9 der Pumpe 2 nach hinten hinausragt. Die Kolbenstange 8 ist hohl ausgebildet und weist einen Entlüftungskanal 10 auf, der den Ansaugraum 11 mit der Außenluft verbindet. An dem in den Ansaugraum 11 mündenden Ende der Kolbenstange 8 ist ein Rückschlagventil 12 zur Entlüftung angeordnet, welches sich beim Ansaughub in Pfeilrichtung 13 schließt. In der der Kolbenstange 8 abgewandten Stirnfläche 14 des Zylinders 9 ist ein ebenfalls als Rückschlagventil ausgebildetes Belüftungsventil 15 vorgesehen, welches über einen Kanal 16 mit dem Milchauffangbehälter 3 und damit mit dem Trichter 1 verbunden ist.

Das hintere freie Ende 17 der Kolbenstange 8 ist verjüngt und als Aufsteckhülse für den Schlauch einer nicht dargestellten Motorpumpe ausgebildet. Der untere Teil 18 des Hebels 5 dient als Handhabe und sein unteres Ende 19 ist als Stütze geformt. Wenn sich der Hebel 5 im gezeichneten Beispiel in einer seiner Endstellungen befindet, dann liegt die Stütze 19 etwa in einer Ebene mit dem Boden 20 des Milchauffangbehälter 3, so daß der Hebel 5 als Abstützvorrichtung für die erheblich über die Grundfläche des Milchauffangbehälters 3 hinausragende Pumpe 2 dient.

Am Trichter 1 ist eine Belüftungsöffnung 21 vorgesehen, die von Hand abgedeckt werden kann und zur Einstellung des richtigen Unterdruckes dient. Um eine Schädigung der Brust durch zu starken Unterdruck mit Sicherheit auszuschließen, ist am Trichter noch ein Rückschlagventil 22 vorgesehen, dessen Federteller verstellt werden kann, so daß eine Einstellung des Höchstdruckes möglich ist. Bei zu starkem Unterdruck kann dann an diesem Ventil zusätzliche Außenluft eintreten.

Zum Absaugen der Milch wird der Trichter 1 an der Brust angesetzt, die Öffnung 21 durch einen Finger verschlossen und die Handhabe 18 des Hebels 5 hin- und herbewegt. Wenn die Handhabe 18 des Hebels 5 in Pfeilrichtung 23, also gegen die Brust gedrückt wird, bewegt sich die Kolbenstange 8 in Pfeilrichtung 13 und nimmt den Kolben 24 mit. Dabei schließt das Entlüftungsventil 12 und öffnet das Belüftungsventil 15, so daß im Trichter ein Unterdruck entsteht. Dabei wird die Milch abgesaugt und läuft in den Behälter 3. Wird die Handhabe 18 in die entgegengesetzte Richtung bewegt, dann schließt das Belüftungsventil 15 und öffnet das Entlüftungsventil 12, so daß der Kolben 24 in seine vordere Stellung bewegt wird. Dabei sind der Trichter 1 und der Milchauffangbehälter 3 durch die Brust und das Ventil 5 nach außen abgeschlossen, so daß der Unterdruck erhalten bleibt, sofern man ihn nicht durch Freigeben der Öffnung 21 ändert.

Sofern mit einer motorisch angetriebenen Pumpe gearbeitet werden soll, muß lediglich ein nicht dargestellter Schlauch dieser Pumpe auf das freie Ende 17 der Kolbenstange 8 aufgeschoben werden. Beim Saugvorgang öffnen sich dann beide Ventile 12 und 15, so daß keine sonstigen Maßnahmen, beispielsweise die Anordnung eines Adapters oder dergleichen erforderlich sind. Zum Aufstellen des Gerätes wird die Handhabe 18 in eine der Endstellungen gebracht und dient dann als Stütze, um das Gewicht der überstehenden Kolbenpumpe 2 aufzufangen.

## Patentansprüche

1. Muttermilchabsaugvorrichtung, bestehend aus einem Trichter (1) und einer daran angeschlossenen, mittels einer Handhabe (18) betätigbaren Kolbenpumpe (2) mit an einer Kolbenstange (8) gehaltenem Kolben (24) sowie einem Entlüftungsventil (12) und einem in der, der Kolbenstange (8) abgewandten Stirnfläche (14) des Pumpenzylinders (9) befindlichen Belüftungsventil (15), wobei beim Ansaughub (13) des Kolbens (24) das Entlüftungsventil (12) schließt und das Belüftungsventil (15) öffnet, während beim Rückhub des Kolbens (24) das Entlüftungsventil (12) öffnet und das Belüftungsventil (15) schließt, dadurch gekennzeichnet, daß die Kolbenstange (8) hohl und als nach außen geführter Entlüftungskanal (10) ausgebildet ist, wobei sich das Entlüftungsventil (12) an der Mündung des Entlüftungskanals (10) in den Ansaugraum (11) befindet.

2. Muttermilchabsaugvorrichtung nach Anspruch 1, dadurch gekennzeichnet, daß die Kolbenstange (8) mit dem einen Ende eines zweiarmigen, schwenkbar (6) gelagerten Hebels (5) gelenkig (7) verbunden ist, dessen anderes Ende (18) die Handhabe bildet.

3. Muttermilchabsaugvorrichtung nach Anspruch 1 oder 2, dadurch gekennzeichnet, daß das nach außen geführte Ende der Kolbenstange (8) als Aufsteckhülse (17) für den Saugschlauch einer Motorpumpe ausgebildet ist und über die Anlenkstelle (7) des Hebels (5) hinausragt.

4. Muttermilchabsaugvorrichtung mit einem Milchauffangbehälter nach Anspruch 2 oder 3, dadurch gekennzeichnet, daß der als Handhabe (18) ausgebildete Hebelteil an seinem freien Ende (19) als Stütze und so lang ausgebildet ist, daß er in mindestens einer Stellung etwa in einer Ebene mit dem Boden (20) des Milchauffangbehälters (3) liegt.

5. Muttermilchabsaugvorrichtung mit einem Ventil zwischen dem Trichter (1) und der Stirnfläche (14) des Pumpenzylinders (9) nach wenigstens einem der vorhergehenden Ansprüche, dadurch gekennzeichnet, daß das Ventil am Trichter (1) angebracht und insbesondere als einstellbares Überdruckventil (22) ausgebildet ist.

## Revendications

1. Appareil pour tirer le lait maternel, comprenant un entonnoir (1) auquel est raccordée une pompe (2) à piston, pouvant être actionnée au moyen d'une poignée (18) et munie d'un piston (24) retenu sur une tige (8), ainsi qu'une soupape de purge (12) et une soupape de ventilation (15) qui est logée dans la face extrême (14) du cylindre de pompage (9), tournée à l'opposé de la tige (8) du piston, la soupape de purge (12) se fermant et la soupape de ventilation (15) s'ouvrant lors de la course d'aspiration (13) du piston (24), tandis que la soupape de purge (12) s'ouvre et la soupape de ventilation (15) se ferme lors de la course de retour du piston (24), caractérisé par le fait que la tige (8) du piston est creuse et est réalisée sous la forme d'un canal d'évent (10) gagnant l'extérieur, la soupape de purge (12) se trouvant à l'embouchure du canal d'évent (10) dans la chambre d'aspiration (11).

2. Appareil pour tirer le lait maternel, selon la revendication 1, caractérisé par le fait que la tige (8) du piston est reliée, de manière articulée (7), à l'une des extrémités d'un levier (5) à deux bras monté pivotant (6), dont l'autre extrémité (18) forme la poignée.

3. Appareil pour tirer le lait maternel, selon la revendication 1 ou 2, caractérisé par le fait que l'extrémité de la tige (8) du piston qui déborde vers l'extérieur est réalisée sous la forme d'un embout d'emboîtement (17) pour le flexible d'aspiration d'une pompe motorisée, et fait saillie au-delà de la zone d'articulation (7) du levier (5).

4. Appareil pour tirer le lait maternel, muni d'un réceptacle collecteur de lait, selon la revendication 2 ou 3, caractérisé par le fait que la partie du levier qui est réalisée sous la forme d'une poignée (18) est conçue en tant que soutien à son extrémité libre (19), et présente une longueur telle qu'elle se trouve, au moins dans une position, sensiblement dans un même plan que le fond (20) du réceptacle (3) collecteur de lait.

5. Appareil pour tirer le lait maternel, présentant une soupape entre l'entonnoir (1) et la face extrême (14) du cylindre de pompage (9), selon au moins l'une des revendications précédentes, caractérisé par le fait que la soupape est installée sur l'entonnoir (1), et est notamment réalisée sous la forme d'une soupape de surpression (22) réglable.

**Claims**

1. A mother's milk extraction device consisting of a funnel (1) and, connected to it and capable of being actuated by a handle (18), a piston pump (2) with a piston (24) supported on a piston rod (8) and with a vent valve (12) and with an air supply valve (15) disposed in the end face (14) of the pump cylinder (9) which is remote from the piston rod (8), and in which the vent valve (12) closes during the induction stroke (13) of the piston (24) while the air supply valve (15) opens, whereas during the return stroke of the piston (24) the vent valve (12) opens and the air supply valve (15) closes, characterised in that the piston rod (8) is hollow and is constructed as an outwardly extended vent passage (10), the vent valve (12) being disposed at the mouth of the vent passage (10) into the induction chamber (11).

2. A mother's milk extraction device according to Claim 1, characterised in that the piston rod (8) is articulatingly connected at (7) to one end of a two-armed lever (5) pivotally mounted at (6) and the other end (18) of which constitutes the handle.

3. A mother's milk extraction device according to Claim 1 or 2, characterised in that the outwardly extending end of the piston rod (8) is constructed as a sleeve (17) onto which it is possible to fit the suction hose of a motor pump and projects beyond the point (7) of articulation of the lever (5).

4. A mother's milk extraction device with a milk receiving container according to Claim 2 or 3, characterised in that the part of the lever which is constructed as a handle (18) is at its free end (19) constructed as a brace and is sufficiently long that at least in one position it lies in the same plane as the bottom (20) of the milk receiving container (3).

5. A mother's milk extraction device with a valve between the funnel (1) and the end face (14) of the pump cylinder (9) according to at least one of the preceding Claims, characterised in that the valve is mounted on the funnel (1) and is in particular constructed as an adjustable over-pressure valve (22).